# EUROPÄISCHE PATENTSCHRIFT

(11) **EP 1 531 723 B1**
(45) Veröffentlichungstag und Bekanntmachung des Hinweises auf die Patenterteilung: **08.04.2009**
(21) Anmeldenummer: 03794936.9
(22) Anmeldetag: 26.08.2003
(51) Int. Cl.: A61B 5/03

(54) **VORRICHTUNG ZUR MESSUNG VON PARAMETERN IM HIRN**
DEVICE FOR MEASURING PARAMETERS IN THE BRAIN
DISPOSITIF POUR MESURER DES PARAMETRES DANS LE CERVEAU

(30) Priorität: 29.08.2002 DE 10239743
(43) Veröffentlichungstag der Anmeldung: 25.05.2005
(73) Patentinhaber: RAUMEDIC AG, 95233 Helmbrechts (DE)
(72) Erfinder: STENDEL, Rüdiger, 14163 Berlin (DE); GÖHLER, Karlheinz, 08297 Zwönitz (DE); KUNZE, Gerd, 08297 Zwönitz (DE); GROPP, Friedrich, 15859 Storkow (DE)
(74) Vertreter: Hofmann, Matthias
(86) Internationale Anmeldenummer: PCT/EP2003/009411
(87) Internationale Veröffentlichungsnummer: WO 2004/023993

(56) Entgegenhaltungen:
- WO-A-02/07596
- US-A- 4 127 110
- US-A- 4 186 749
- US-A- 4 281 667
- US-A- 4 519 401

## Beschreibung

Es ist bekannter Stand der Technik für die Messung des Hirndruckes, wie anderer Parameter im Hirn des Menschen, Sonden epidural oder subdural zu implantieren.
Diese Sonden sind mit Messsensoren bestückt, die den Hirndruck erfassen, in elektrische Signale umwandeln und über eine Kabelverbindung an einen Patientenmonitor leiten.
Dort werden die Messwerte verarbeitet und in Form von Zahlenwerten und grafisch als Kurven dargestellt.
Die Kabelverbindungen zwischen Sonden mit Messsensoren und den Patientenmonitoren sind jedoch nur sehr aufwändig herzustellen, da die Patientenmonitore verschieden gestaltete Steckerbuchsen aufweisen und fehleranfällig in der Handhabung sind. Zudem erfordert fast jedes Diagnosemesselement ein spezielles Kabel, so dass vor allem auf der Intensivstation Patienten mit einer unübersichtlichen Menge an Kabeln verbunden sind, was zu Komplikationen in der Pflege führt und ein Risiko für den Patienten darstellt.
Dies trifft besonders dann zu, wenn der Patient in Stresssituationen behandelt werden muss oder transportiert wird.
Fehlmessungen oder der Totalausfall der Messsonden sind unter Umständen möglich, mit der Konsequenz, nochmals neue Messsonden implantieren zu müssen.
Insgesamt ist also die Verwendung von Kabelverbindungen teuer und im Einzelfall mit hohen Risiken für den Patienten verbunden.
Derartige Sonden mit integrierten Messsensoren zur Implantation stellen beispielsweise die Firmen REHAU AG + Co, Johnson & Johnson, Camino, Medtronic her.

Die WO-A-02/07596 beschreibt eine Vorrichtung zur Messung von Hirnparametern. Eine Sensoreinheit dieser Messvorrichtung ist distal im Parenchym oder den Ventrikeln implantiert. Verschiedene Befestigungseinheiten für die Sensoreinheit sind beschrieben. Die Messvorrichtung umfasst zusätzlich eine Elektronikeinheit.

Gegenstand der DE 43 29 898 A1 ist ein kabelloses, medizinisches Diagnose- und Überwachungsgerät, beispielsweise auch für Neuromonitoring. Die Vorrichtung umfasst eine Auswertestation und eine oder mehrere, an der Hautoberfläche des Patienten angebrachte, Elektroden.
Die Elektroden umfassen eine digitale Sendeeinheit mit Antenne, ggf. eine Empfangseinheit, eine Energieversorgungseinheit sowie mindestens einen Halbleitersensor. Die Halbleitersensoren können u. a. zur Detektion von EEG- oder EKG-Signalen verwendet werden.
Nachteilig an dieser Lösung ist, dass ausschließlich Elektroden verwendet werden können, welche an der Hautoberfläche des Patienten angebracht sind.

Der Versuch in Shunt-Systemen zur Behandlung von Hydrocephali Hirndruckmessungen dauerhaft oder ambulant zu realisieren, führte zur Kombination von implantierten Messsonden mit Sensoren, deren Messsignale telemetrisch mit der jeweiligen Auswerteeinheit verbunden sind.
Beispielsweise beschreibt DE 197 05 474 A1 eine implantierbare Messeinheit zur Messungen u. a. von Hirndrücken. Dabei sind Sensorelement und Telemetrieeinheit auf einer flexiblen Folie aufgebracht. Die Telemetrieeinheit hat eine äußere Spule, über welche die implantierte Schaltung induktiv mit Energie versorgt wird, außerdem werden dabei induktiv die im Senderelement gemessenen Daten an die Auswerteeinheit übertragen.
Nachteilig ist, dass eine solche induktive, kabellose Übertragung von Daten oder Energie nur über eine sehr kurze Distanz - einige Millimeter - funktioniert, also nur epidurale, evtl. auch subdurale Messungen möglich sind. In der DE 43 41 903 A1 wird eine besonders kleine, implantierbare Vorrichtung beschrieben, deren Außenabmessungen kleiner 1,0 x 1,5 x 0,6 mm sind und die zur kontinuierlichen Messung des Druckes und/oder des Durchflusses und/oder der Temperatur in Körpern oder Organen von Menschen oder Tieren geeignet ist. Diese Vorrichtung übermittelt Netzwerte bzw. Messsignale ohne Verkabelung perkutan an einen außerhalb des Körpers befindlichen Empfänger, welcher die Messsignale verarbeitet und zur Anzeige bringt.
Solche auf einem Chip integrierten, also fest gekoppelten Systeme Sensor - Telemetrieeinheit sind nicht geeignet, die gewünschten Parameter (beispielsweise Himdruck, Temperatur) an den für die Indikationsstellung optimalen Orten zu messen.
Problemlos lassen sie sich nämlich ausschließlich epidural, evtl. auch subdural, implantieren. Ihre Implantation in die für die Messungen wesentlich günstigeren Orte, nämlich in das Parenchym oder die Ventrikel, ist nicht möglich.

In diesen Bereichen ist auch die externe Energieversorgung durch Induktion oder HF-Felder kaum noch möglich, damit ist die Funktion der Mess- und Sendeeinheit nur für kurze Zeit gegeben.
Weiterhin führt der oft nötige, zusätzliche Einsatz bildgebender Verfahren, wie die Magnetresonanz, zur Funktionsstörung der implantierten Steuer- und Regelungstechnik bzw zu induktiven Strömen im Schaltungssystem, sowie nicht zuletzt zur Erwärmung und Schädigung des die Implantate umgebenden Gewebes.

Generell ist zum beschriebenen Stand der Technik bei der telemetrischen Übertragung von Signalen aus implantierten Sensoren zu sagen, dass bisher keine Berichte über deren erfolgreiche, praktische Umsetzung vorliegen.

Da für die korrekte Erfassung von physiologischen Daten im menschlichen Hirn neben der Auslegung der sensitiven und spezifischen Sensoren vor allem die Messorte entscheidend sind, stellte sich daher die Aufgabe der vorliegenden Erfindung, eine Vorrichtung zur Messung von Parametern im Hirn bereitzustellen, die über folgende Eigenschaften verfügt:
- Die Messung der gewünschten Parameter ist an den üblichen = klassischen, medizinisch anerkannten Stellen, nämlich im Parenchym und/oder in den Ventrikeln möglich; bei Bedarf sollten auch weiterhin die epiduralen oder subduralen Messungen möglich sein.
- Die Übertragung und Aufbereitung der Patientendaten erfolgt digital und via Telemetrie.
- Es steht ein Baukastensystem zur Verfügung, mit dem die Messvorrichtung - je nach Anforderung - maßgeschneidert zusammengestellt werden kann.
- Die Elektronik-Einheit ist nach ihrer Sterilisation wieder verwendbar.

Die Aufgabe konnte erfindungsgemäß dadurch gelöst werden, dass
- die Sensoren in einem Katheter aus polymeren Werkstoffen angeordnet sind, der ggf. mindestens über ein Lumen zur Liquordrainage verfügt
- die Elektronikeinheit in einer abgeschlossenen, vorzugsweise ringförmig angeordneten Baugruppe aufgenommen ist
- der Katheter in der mittigen Ausnehmung der Grundplatte über ein ringförmiges Befestigungselement fest und dicht, jedoch lösbar fixiert ist
- Sensoreinheit und Elektronikeinheit über einen Mikrostrecker verbunden sind
- die Messeinheit mit Katheter und Sensor, der auf der Grundplatte montierten Elektronikeinheit mit der Energieversorgung und darüber eine wieder lösbare Abdeckung, komplett unter der Kopfhaut auf dem Schädelknochen platziert und nach außen vollständig abgeschlossen ist.

### Nachfolgend soll nun die Erfindung näher beschrieben werden:

Die Grundplatte ist semiflexibel, sie verfügt über eine mittige Ausnehmung mit Stutzen und integriertem, ringförmigem Befestigungselement. Alternativ kann auch an dessen Stelle ein Kugelgehäuse mit Ventil vorgesehen werden, das für Katheter mit wenigstens zwei verschiedenen Größen geeignet ist und das auch den Schrägsitz des Katheters in der Grundplatte ermöglicht.
Die Grundplatte wird nach ihrer Komplettierung mit der Elektronikeinheit und dem Katheter zur Implantation mit einer flexiblen, dichten und wieder lösbaren Abdeckung versehen.

Die Sensoreinheit besteht aus einem Katheter mit einem oder mehreren Sensoren zur Messung beispielsweise von Hirndruck, Temperatur, CO₂-Sättigung oder pH-Wert usw. Zur Liquordrainage kann auch mindestens ein Lumen im Katheter integriert sein. Der Katheter verfügt am proximalen Ende über einen Mikrostecker, der den Kontakt zur Elektronikeinheit herstellt, so dass die Messsignale erfasst und an die Auswerteeinheit weitergegeben werden können.

Die unter der semiflexiblen Abdeckung angeordnete Elektronikeinheit ist nach der Demontage von der Grundplatte und der Abkopplung des Katheters durch Lösen des Mikrosteckers resterilisierbar und damit wieder verwendbar.

Besonders vorteilhaft an der erfindungsgemäßen Vorrichtung ist, dass auf Grund des modularen Aufbaus die Teilelemente je nach der aktuellen Anwendung zusammengestellt werden können. So lässt sich beispielsweise für die Messung im Parenchym ein kurzer Katheter mit einem Durchmesser von 3 CH, für Messungen im Ventrikelbereich mit Liquordrainage ein kurzer Katheter mit einem Durchmesser von 6 CH einsetzen.

Erfindungsgemäß ist weiterhin besonders vorteilhaft, dass Sensoreinheit und Elektronikeinheit zunächst voneinander getrennt sind. Daher lässt sich der den Sensor / die Sensoren enthaltende Katheter auf übliche Weise, beispielsweise - nach Öffnung der Kopfhaut und Setzen eines Bohrlochs im Schädelknochen - über Hülse und Mandrin minimalinvasiv an den optimalen Messorten, nämlich den Ventrikeln oder dem Parenchym, platzieren.
Anschließend wird das proximale Ende des Katheters mit der mittigen Ausnehmung der Grundplatte über das Befestigungselement dicht verschraubt und mit der Elektronikeinheit über den Mikrostecker verbunden. Schließlich wird die so komplettierte Grundplatte mit einer semiflexiblen Abdeckung dicht verbunden und die Kopfhaut wieder geschlossen.

Die Einbettung des Sensors und der Verbindungsleitung in einen Katheter und in ein nicht metallisches Sensorgehäuse verhindert die Erwärmung des umliegenden Gewebes sowie die Dislokation am Messort und damit das Auftreten von Artefakten bei der Messung und der Anwendung der bildgebenden Diagnostik, insbesondere in der magnetischen Resonanztomografie (MRT).
Im Falle der Verwendung von Akkus ist so eine induktive, thermoelektrische oder HF-Feld-Aufladung gewährleistet. Deren Funktion kann durch eine Abschirmung der Bauteile oder durch das Abschalten der Sensoreinheit während der MRT-Untersuchung geschützt werden.

Im Falle von Kathetern, die über Messsensoren und ein Lumen zur Liquorableitung verfügen, ist auf der Grundplatte ein Verbindungsstutzen integriert, der das Lumen aus der Messeinheit patientennah ableitet und an einen Katheter, der in den Brust- oder Bauchraum des Patienten führt, ankoppelt. Möglich ist auch die Verbindung zu einem Shuntventil.
Jeder Systemaufbau lässt sich daher grundsätzlich als geschlossenes System gestalten.

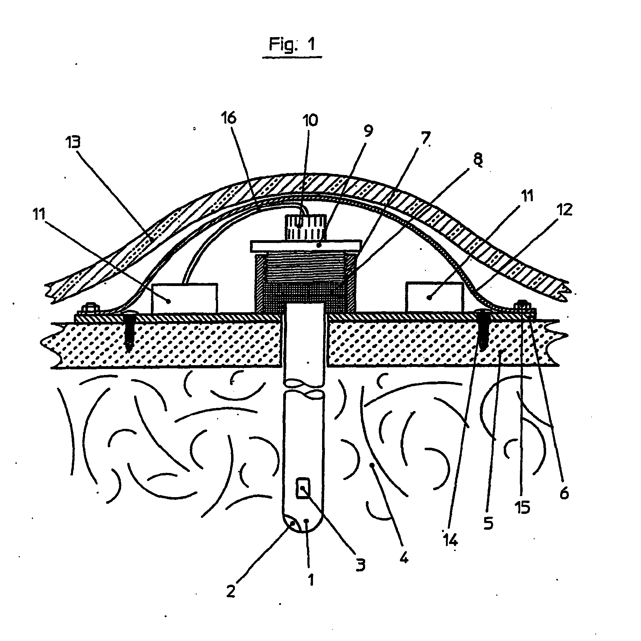

Eine interessante, erfindungsgemäße Möglichkeit zur Energieversorgung des implantierten Systems ist folgende:
Wird der Hirndruck im Ventrikel gemessen, lässt sich der Liquorfluss zur Energiegewinnung mittels eines miniaturisierten Dynamos heranziehen. Dabei ist im Sensorgehäuse eine Kammer mit Ein- und Ausströmöffnung integriert, zwischen denen eine Turbine mit angeschlossenem Dynamo montiert ist.
In einem Ausführungsbeispiel soll nun die Erfindung im Detail erläutert werden; ergänzend dazu siehe die zeichnerische Erläuterung in Fig. 1.

### Ausführungsbeispiel:

Die implantierte, modular aufgebaute Vorrichtung besteht aus einem Katheter 1, der an seinem distalen Ende über einen Temperatursensor 2 und einen Drucksensor 3 verfügt und durch den Schädelknochen 5 ins Hirngewebe 4 hineinragt.
Die auf dem Schädelknochen 5 mittels Schraube 14 befestigte Grundplatte 6 umfasst eine Elektronikeinheit und ein integriertes Befestigungselement 7 mit innenliegendem Gewinde. Über dieses Gewinde bringt die Schraube 9 eine Kraft auf die Dichtung 8 auf, wodurch der Raum zwischen der semiflexiblen Abdeckung 12 und der Grundplatte 6 dicht gegenüber dem Hirngewebe 4 abgeschlossen ist, gleichzeitig der Katheter 1 auf der Grundplatte 6 und damit dem Schädelknochen 5 fixiert ist.
Der auf dem proximalen Ende des Katheters 1 befindliche Mikrostecker 10 ist über eine Leitung 16 mit der Elektronikeinheit 11 verbunden.
Über ein Funksignal wird nun die Vorrichtung auf Funktion geprüft. Anschließend wird mittels der Schrauben 15 die semiflexible Abdeckung 12 dicht, jedoch wieder lösbar, mit der Grundplatte 6 verbunden.
Die Kopfhaut 13 überspannt und schützt die implantierte Vorrichtung.

Die Elektronikeinheit 11 kann durch Verwendung von Thermoelementen oder piezoelektronischen Vorrichtungen oder Nanoturbinen im Liquorraum körpereigene Energien nutzen.

## Patentansprüche

1. Vorrichtung zur Messung von Hirnparametern
- mit einer Sensoreinheit (1-3), die derart ausgeführt ist, dass sie
-- distal minimalinvasiv im Parenchym und/oder den Ventrikeln implantierbar ist,
-- proximal in einem auf einer Grundplatte (6) zentriert angeordneten Befestigungselement (7) aufgenommen ist,
**dadurch gekennzeichnet, dass**
- die Sensoreinheit (1-3) mit einer Elektronikeinheit (11) über einen Mikrostecker (10) elektrisch leitend verbunden ist,
- die Messvorrichtung eine proximale Baugruppe aufweist, umfassend:
-- das Befestigungselement (7),
-- die im Befestigungselement (7) aufgenommenen Abschnitte der Sensoreinheit (1-3),
-- die Elektronikeinheit (11),
-- den Mikrostecker (10),
- wobei die proximale Baugruppe (1-3, 7, 10, 11) derart ausgeführt ist, dass sie mittels einer semiflexiblen Abdeckung (12) fest und dicht, jedoch wieder lösbar, angeschlossen und zwischen Schädelknochen und Kopfhaut positionierbar ist.

2. Vorrichtung nach Anspruch 1, **dadurch gekennzeichnet, dass** das Gesamtsystem modular aufgebaut ist.

3. Vorrichtung nach Anspruch 1, **dadurch gekennzeichnet, dass** die Elektronikeinheit (11) aus den Hauptkomponenten Energieversorgung, Sender, Empfänger, Steuereinheit und Mikrosteckerbuchse zusammengesetzt ist.

4. Vorrichtung nach Anspruch 1, **dadurch gekennzeichnet, dass** die Sensoreinheit umfasst einen Katheter (1) aus polymerem Werkstoff, sowie einen oder mehreren Sensoren (2, 3) zur Messung von Hirndruck und/oder Temperatur und/oder CO₂-Partialdruck und/oder Sauerstoffpartialdruck.

5. Vorrichtung nach Anspruch 4, **dadurch gekennzeichnet, dass** der Katheter (1) mindestens ein Lumen für die Sensorik, ggf. zusätzlich mindestens ein Lumen für die Liquordrainage enthält.

6. Vorrichtung nach einem der Ansprüche 1 oder 5, **dadurch gekennzeichnet, dass** der ein Lumen für die Liquordrainage enthaltende Katheter (1) über einen Stutzen der Grundplatte (6) an einen weiteren, im Bauchraum des Patienten verlegten Katheter angeschlossen wird und mit diesem ein geschlossenes System bildet.

7. Vorrichtung nach einem der Ansprüche 1 oder 3, **dadurch gekennzeichnet, dass** die Elektronikeinheit (11) sterilisierbar und wieder verwendbar ist.

8. Vorrichtung nach den Ansprüchen 1, 3, oder 7, **dadurch gekennzeichnet, dass** die Elektronikeinheit (11) körpereigene Energien nutzt durch Verwendung von Thermoelementen oder piezoelektronischen Vorrichtungen oder Nanoturbinen im Liquorraum.

## Claims

1. Device for measuring brain parameters,
- comprising a sensor unit (1-3) which is designed in such a way that
-- it is distally implantable minimally invasively in the parenchyma and/or the ventricles,
-- it is received proximally in a fastening element (7) that is disposed centrally on a base plate (6),
**characterized in that**
- the sensor unit (1-3) is electrically conductively connected to an electronic unit (11) by means of a micro plug (10),
- the measuring device comprises a proximal assembly comprising:
-- the fastening element (7);
-- the portions of the sensor unit (1-3) which are received in the fastening element (7);
-- the electronic unit (11);
-- the micro plug (10);
the proximal assembly (1-3, 7, 10, 11) being designed in such a way that it is rigidly and tightly but removably connected by means of a semi-flexible cover (12) and positionable between skull bone and scalp.

2. Device according to claim 1, **characterized in that** the overall system is modularly designed.

3. Device according to claim 1, **characterized in that** the electronic unit (11) is composed of the main components power supply, transmitter, receiver, control unit and micro-plug socket.

4. Device according to claim 1, **characterized in that** the sensor unit comprises a catheter (1) of polymeric material, as well as one or more sensors (2, 3) for measuring brain pressure and/or temperature and/or CO₂ partial pressure and/or oxygen partial pressure.

5. Device according to claim 4, **characterized in that** the catheter (1) contains at least one lumen for the sensor system, optionally at least one additional lumen for the drainage of cerebrospinal fluid.

6. Device according to any of claims 1 or 5, **characterized in that** the catheter (1) that contains a lumen for the drainage of cerebrospinal fluid is connected, by means of a connection piece of the base plate (6), to an additional catheter placed in the patient's abdominal cavity and together with it forms a closed system.

7. Device according to any of claims 1 or 3, **characterized in that** the electronic unit (11) is sterilizable and reusable.

8. Device according to any of claims 1, 3 or 7, **characterized in that** the electronic unit (11) uses endogenous energies through utilization of thermocouples or piezoelectronic devices or nanoturbines in the subarachnoid space.

## Revendications

1. Dispositif de mesure de paramètres cérébraux
- avec une unité à capteur (1-3) exécutée de manière
-- à être implantable distalement de manière mini-invasive dans le parenchyme et / ou les ventricules,
-- à être logée proximalement dans un élément de fixation (7) disposé centré sur une plaque de base (6),
**caractérisé en ce que**
- l'unité à capteur (1-3) est reliée de manière électriquement conductive à une unité électronique (11) par une microfiche (10),
- le dispositif de mesure comprend un module proximal comprenant :
-- l'élément de fixation (7),
-- les parties de l'unité à capteur (1-3) logées dans l'élément de fixation (7)
-- l'unité électronique (11),
-- la microfiche (10),
- le module proximal (1-3, 7, 10, 11) étant réalisé de manière à être raccordé fixement et de manière étanche, mais en restant toujours amovible, au moyen d'une couverture semi-flexible (12), et à être positionnable entre les os crâniens et le cuir chevelu.

2. Dispositif selon la revendication 1, **caractérisé en ce que** le système d'ensemble est modulairement structuré.

3. Dispositif selon la revendication 1, **caractérisé en ce que** l'unité électronique (11) est formée des composants principaux que sont une alimentation en courant, un émetteur, un récepteur, une unité de commande et une prise de microfiche.

4. Dispositif selon la revendication 1, **caractérisé en ce que** l'unité à capteur comprend un cathéter (1) en matériau polymère, ainsi qu'un ou plusieurs capteurs (2, 3) pour la mesure de la pression cérébrale et / ou de la température et / ou de la pression partielle de CO₂ et / ou de la pression partielle d'oxygène.

5. Dispositif selon la revendication 4, **caractérisé en ce que** le cathéter (1) comprend au moins un lumen pour le dispositif à capteur, et le cas échéant au moins un lumen en complément pour le drainage de liquide céphalo-rachidien.

6. Dispositif selon la revendication 1 ou la revendication 5, **caractérisé en ce que** le cathéter (1) comprenant un lumen pour le drainage de liquide céphalo-rachidien est relié par un raccord de la plaque de base (6) à un autre cathéter posé dans la cavité abdominale du patient, et forme un système fermé avec celui-ci.

7. Dispositif selon l'une des revendications 1 ou 3, **caractérisé en ce que** l'unité électronique (11) est stérilisable et réutilisable.

8. Dispositif selon les revendications 1, 3 ou 7, **caractérisé en ce que** l'unité électronique (11) exploite des énergies corporelles spécifiques en utilisant des éléments thermiques ou des dispositifs piézo-électriques, ou des nanoturbines dans l'espace subarachnoïde.
